**Europäisches Patentamt**

**European Patent Office**

**Office européen des brevets**

(19)

(11) Veröffentlichungsnummer: **0 259 252 B1**

## (12) EUROPÄISCHE PATENTSCHRIFT

(45) Veröffentlichungstag der Patentschrift: 06.03.91

(51) Int. Cl.⁵: **G01N 33/44**, G01N 25/04

(21) Anmeldenummer: 87810453.8

(22) Anmeldetag: 10.08.87

(54) **Verfahren und Vorrichtung zur Ermittlung des zeitlichen Gelierverhaltens eines reaktiven Harzsystemes.**

(30) Priorität: 14.08.86 CH 3258/86

(43) Veröffentlichungstag der Anmeldung:
09.03.88 Patentblatt 88/10

(45) Bekanntmachung des Hinweises auf die
Patenterteilung:
06.03.91 Patentblatt 91/10

(84) Benannte Vertragsstaaten:
**AT CH DE FR GB IT LI**

(56) Entgegenhaltungen:
**EP-A- 0 169 252**
**DE-A- 2 612 253**
**DE-A- 2 829 450**
**US-A- 4 164 136**
**US-A- 4 512 182**

(73) Patentinhaber: **CIBA-GEIGY AG**
**Klybeckstrasse 141**
**CH-4002 Basel(CH)**

(72) Erfinder: **Poll, Dietmar**
**Neumatt 254**
**CH-1711 St. Silvester(CH)**

## Beschreibung

Die Erfindung betrifft ein Verfahren und eine entsprechende Vorrichtung zur Ermittlung des zeitlichen Gelierverhaltens eines reaktiven Harzsystems gemäss dem Oberbegriff des Anspruchs 1 und dem Oberbegriff des Anspruchs 6.

In der Kunststoffverarbeitung und -forschung stellt die Messung der Gelierzeit (B-Zeit) von Reaktionsharzen ein bisher sehr oft behandeltes Problem dar. Charakteristische Einflussgrössen dabei sind Probengrösse (Masse), Probendimension (dick, flach), Aufheizung, Temperierung, Exothermieabfuhr, Definition des Gelierpunktes, Zeitnehmung, offene Oberfläche (Lösungsmittelabdampfung). Wärmeabfuhr durch Strahlung, Leitung und Konvektion und schliesslich der Einfluss der versuchsdurchführenden Person.

Eine bekannte Vorrichtung zur Gelierzeitbestimmung ist die sog. Gelierzeitplatte. Dabei wird auf einer geheizten Platte ein mehr oder weniger dünner Film der Probe aufgebracht und mit einer Holzspachtel oder Drahtklammer in gewissen Zeitabständen kontrolliert, ob die Probe Fäden zieht. Wird der gummielastische Zustand erreicht (räumliche Vernetzung), spricht man vom Gelpunkt. Die Gelierplatte ist konstruktiv einfach und benötigt keine aufwendige Probenvorbereitung. Durch die grosse Oberfläche können aber flüchtige Anteile (Lösungsmittel) sehr schnell verdampfen und dadurch die Reaktionsgeschwindigkeit beeinflussen, was die Resultate verfälscht.

Weitere Nachteile bestehen in der nicht homogenen Temperaturverteilung auf solchen Platten (Unterschiede von $\pm 3^{\circ}$ C), in der grossen Wärmeabstrahlung und Konvektion, im Nichterfassen des Temperaturverlaufs während der Aufheizung bzw. Exothermie, in der Wärmeableitung durch Spachtel und im Einfluss der versuchsdurchführenden Person durch Anzahl und Richtung der Striche je Zeiteinheit.

Die mit dieser Vorrichtung gemessenen Werte zeigen oberhalb einer gewissen Temperatur (z.B. $160^{\circ}$ C, je nach Harzsystem) eine starke Abweichung zu grösseren Zeiten im Arrheniusdiagramm, die durch verursacht sind, dass die Reaktion bis zur Gelierung bereits in der Aufheizphase abgewschlossen ist. Bisher wurden diese Werte ignoriert und die Arrheniusgerade nach Gefühl zwischen einen meist weit gestreuten Punktehaufen gelegt. Es können auch nicht mit Sicherheit latente Systeme bzw. der Knickpunkt im Arrheniusdiagramm ermittelt werden. Messfehler von 100 %; und mehr bei höheren Temperaturen sind keine Seltenheit.

Ein weiteres bekanntes Gelierzeitmessgerät besteht aus einer Gelierzeitplatte mit zwei Vertiefungen, in denen kleine Drahtbügel hin- und herbewegt werden. Diese Drahtbügel bewirken bie Gelierung des Harzes in diesen Vertiefungen über Druckschalter das Ausschalten des Antriebes und der Stoppuhr. Die Vorteile dieses Gerätes sind sein einfacher Aufbau und der weitgehend automatische Messablauf. Es besitzt jedoch im wesentlichen dieselben Nachteile wie die Gelierplatte. Ferner entstehen durch die Kinematik des Antriebes des Drahtbügels (Nockentrieb) in den Wendepunkten lange Pausen, in denen keine Messung erfolgen kann. In der Folge muss der Schalter einen relativ grossen Weg zurücklegen, um die Uhr und den Antrieb auszuschalten. Befindet sich der Drahtbügel ca. 2 mm vor dem Wendepunkt und tritt in diesem Moment die Gelierung ein, wird eine um 20 sec zu hohe Gelierzeit gemessen, weil der Schalter die 2 mm bis zum Umkehrpunkt und zurück und zusätzlich 2 mm bis zur Auslösung zurücklegen muss. Bei höheren Temperaturen betragen die Gelierzeiten sehr oft weniger als 1 Minute und hier ist ein solcher Messfehler untragbar. Die Probendicke beträgt bei diesem Gerät ca. 5 mm und die Oberfläche ist weitgehend frei (nur durch ein etwa 5 mm über der Probe angebrachtes Blech geschützt). Durch Abstrahlung und Konvektion entsteht über den Querschnitt der Probe ein Temperaturprofil mit einem Unterschied von bis zu $10^{\circ}$ C. Es kann nun vorkommen, dass die Gelierzeiten zwischen Ober- und Unterseite der Probe stark voneinander abweichen, das heisst, die Unterseite ist bereits vollständig gehärtet, während die Oberseite noch flüssig ist. Es kommt nun sehr darauf an, auf welcher Höhe sich der Bügel in der Probe bewegt. In manchen Fällen schneidet dieser Bügel auch noch das angelierte Material durch und schaltet bei Gelierung nicht ab.

Ein weiteres bekanntes Gerät ist das sog. TECAM-Messgerät. Dieses Gerät besitzt einen durch Kurbeltrieb auf- und abbewegten Stift, an dessen unterem Ende ein Teller mit ca. 25 mm Durchmesser befestigt ist. Dieser Teller wird in das Harz eingetaucht; er bleibt bei Gelierung stecken. Ueber einen Druckschalter, der nur bei Abwärtsbewegung wirksam ist, werden Motor und Uhr abgestellt. Dieses Verfahren ist für sehr lange Gelierzeiten (mehrere Stunden) und am besten bei Raumtemperatur geeignet. Das Gerät ist relativ einfach und erlaubt einen vollautomatischen Betrieb. Seine Nachteile sind lange Aufheizzeiten, Verfälschung der Messwerte durch Exothermie, grosse Probenmengen, sinusförmige Geschwindigkeit des Prüfkörpers und andauernde Störung des Molekülaufbaues durch den Teller. Ferner beträgt die Umlaufzeit der Kurbel 1 min, wobei in der Aufwärtsbewegung während $\frac{1}{2}$ min und in den Umkehrpunkten 2 mal 5 sec die Uhr nicht abgeschaltet werden kann. Der maximale Fehler beträgt also 40 sec. Bei bekannter oder gut abzuschätzender Gelierzeit kann allerdings die Position der Kurbel beim Start so gewählt werden, dass der voraussichtliche Schaltpunkt in der Abwärtsbewegung liegt. Dadurch kann dieser Messfehler vermieden werden.

Eine weitere Methode zur Gelierzeitbestimmung ist die nach DIN 16945. Dabei wird in einem lose aufgehängten harzgefüllten Proberöhrchen mit Durchmesser 20 mm ein Glasstab mit einer Verdickung am unteren Ende oder eine Metallspirale in einem bestimmten Zyklus auf- und abbewegt. Bei Gelierung bleibt der Stab im Harz stecken und zieht das Röhrchen mit nach oben. Dadurch wird ein Schalter betätigt, der eine Uhr abstellt. Dieses Verfahren wird nur für ungesättigte Polyester empfohlen, da bei UP-Harzen der Gelierpunkt vor der exothermen Reaktion liegt und somit der Fehler durch Exothermie nicht ins Gewicht fällt.

Diese Methode ist nur bei Systemen mit wenig Exothermie oder mit Gelierpunkt vor der exothermen Reaktion einsetzbar, erfordert dicke Proben, dadurch lange Aufheizzeiten, und erlaubt das Abschalten nur in einer Richtung. Bei der normgemässen Vorrichtung ist noch die Möglichkeit vorgesehen, die Temperatur während der Reaktion zu verfolgen. Es ist jedoch nicht entnehmbar, welchem Zweck dies dienen und wie die Temperaturkurve ausgewertet werden soll. Es wäre auch nicht sehr sinnvoll, die gemessene Temperatur als (mittlere) Massetemperatur anzusehen, denn in so dicken Proben herrscht immer ein beträchtliches Temperaturprofil.

Es existieren noch einige andere Gelierzeitbestimmungsgeräte, die mit DSC- (Differential Scanning Calorimetry-) und DTA- (Differential Thermal Analysis-) Methoden arbeiten, jedoch lässt der Wärmehaushalt einer Reaktion keine eindeutigen Rückschlüsse auf den Gelierpunkt zu. Auch elektrische Widerstands- und Kapazitätsmessungen haben bisher noch nicht zuverlässige Aussagen erlaubt. In einem weiteren bekannten Verfahren werden die beiden Komponenten zuerst auf die Solltemperatur vorgewärmt und dann gemischt. Dies ist aber nur bei mässig schnellen Systemen sinnvoll. Die Exothermie wird dabei auch nicht berücksichtigt.

Es gibt auch Publikationen, in denen der Gelierpunkt bei einem bestimmten chemischen Umsatz festgelegt wird, dieser Umsatz ist aber von System zu System unterschiedlich (vgl. Ungesättigter Polyester-Epoxidharz).

Davon ausgehend sollen durch die Erfindung die verfahrensmässigen und apparativen Voraussetzungen für eine verbesserte Untersuchungsmöglichkeit des Gelierverhaltens geschaffen werden, bei dem die Einflüsse möglichst vieler der eingangs angeführten Grössen minimiert oder wenigstens quantifizierbar gemacht werden. Mit anderen Worten, es sollen ein genaueres, präziseres, zweckmässigeres und signifikantere Ergebnisse lieferndes Verfahren und eine entsprechende Vorrichtung geschaffen werden, welche zudem noch die Möglichkeit der Automatisierung bieten.

Diese der Erfindung zugrundeliegende Aufgabe ist durch das im Anspruch 1 definierte Verfahren und die im Anspruch 6. definierte Vorrichtung gelöst. Bevorzugte Ausgestaltungen und Weiterbildung sind in den abhängigen Ansprüchen beschrieben.

Im folgenden wird die Erfindung anhand der Zeichnung näher erläutert: Es zeigen:

Fig. 1    in schematischer Darstellung ein Ausführungsbeispiel einer erfindungsgemässen Vorrichtung,

Fig. 2    einen typischen zeitlichen Temperaturverlauf eines Harzsystems während der Gelierung,

Fig. 3    Temperaturverläufe bei verschiedenen Solltemperaturen, und

Fig. 4    ein typisches Arrhenius-Diagramm nach herkömmlichen Messmethoden.

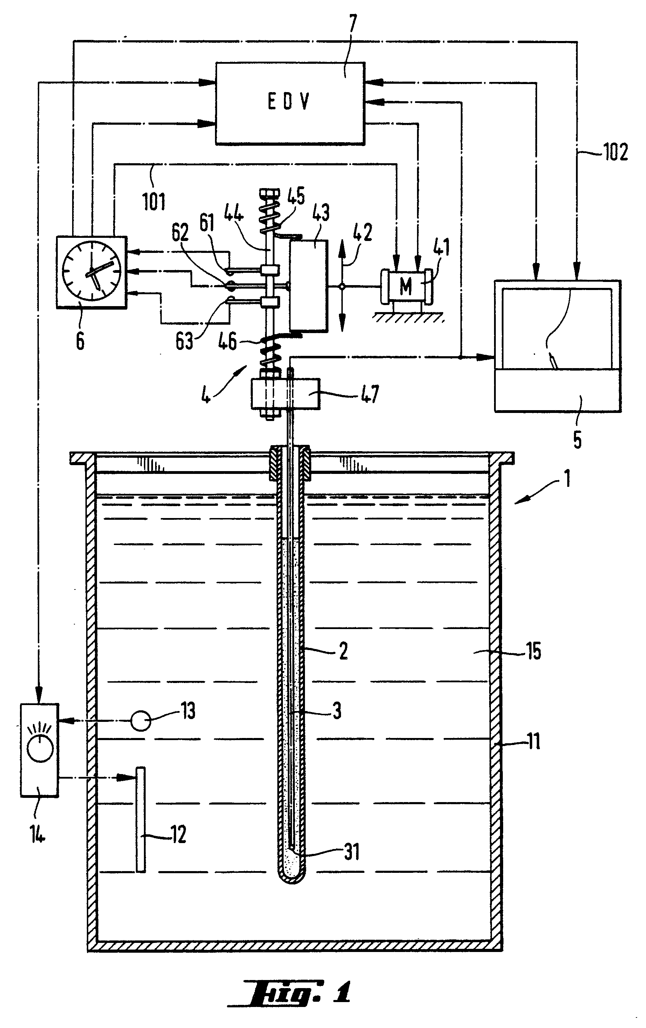

Gemäss Fig. 1 umfasst die erfindungsgemässe Vorrichtung ein Heizorgan in Form eines thermostatisch geregelten Heizbades 1, ein rohrförmiges Behältnis 2 für das zu untersuchende Harzsystem, einen Temperaturfühler 3 für die Temperatur des im Behältnis befindlichen Harzsystems, einen als Ganzes mit 4 bezeichneten Antrieb zum Auf-und Abbewegen des Temperaturfühlers im Behältnis, ein an den Temperaturfühler angeschlossenes Registriergerät 5 für den zeitlichen Temperaturverlauf des untersuchten Harzsystems, eine Stoppuhr 6 zur Erfassung der bis zum Einsetzen der Gelierung vergangenen Zeit und eine elektronische Datenverarbeitungseinrichtung 7 zur automatischen Auswertung der erfassten Temperatur- und Zeitdaten.

Hier und im folgenden wird unter "Harzsystem" ein reaktives Gemisch aus Harz, Härter, allfälligen Beschleunigern, allfälligen Füllstoffen und allfälligen weiteren Komponenten verstanden.

Das Heizbad 1 umfasst einen gegenüber dem rohrförmigen Behältnis 2 grossvolumigen Behälter 11, in dem sich ein elektrischer Heizstab 12 und ein Temperaturfühler 13 befinden, welche an eine Temperaturregelung 14 angeschlossen sind. Das im Heizbad befindliche Wärmeübertragungsmedium 15 ist üblicherweise ein temperaturfestes Oel. Die Badtemperatur ist wahlweise einstellbar und wird von der Regelung konstant gehalten.

Der Antrieb 4 ist in nicht gezeigter Weise ortsfest oberhalb des Heizbades 1 angeordnet. Er umfasst einen Motor 41, der über hier nur durch den Doppelpfeil 42 symbolisierte Getriebeteile eine auf- und abgehende Bewegung eines Treibelementes 43 bewirkt. Dieses ist über zwei durch einen vertikalen Stab 44 miteinander verbundene Koppelfedern 45 und 46 kinematisch mit dem Stab 44 und einer an diesem befestigten Bride 47 verbunden, in welcher der Temperaturfühler 3 lösbar eingespannt ist. Die Auf- und Abbewegung des Treibelements 43 überträgt sich somit über die Koppelfedern 45 und 46, den Stab 44 und die Bride 47 auf den Temperaturfühler 3.

Am Stab 44 sind im gegenseitigen Abstand zwei Schaltkontakte 61 und 62 befestigt. In der Mitte zwischen diesen beiden Kontakten befindet sich ein weiterer Schaltkontakt 63, der am Treibelement 43 befestigt ist und von diesem mitbewegt wird. Die drei Schaltkontakte 61-63 sind an die Stoppuhr 6 angeschlossen.

Der motorisch auf- und abbewegte Temperaturfühler 3 mit seinem federgekoppelten Antrieb und die Schaltkontakte 61-63 bilden zusammen eine Viskositätsmesseinrichtung für das im Probenröhrchen 2 befindliche Harzsystem. Solange das Harzsystem noch relativ dünnflüssig ist, setzt es der Bewegung des Temperaturfühlers 3, der hier gleichzeitig die Funktion eine Viskositätsfühlers hat, keinen nennenswerten Widerstand entgegen. Der Stab 44 wird also der Bewegung des Treibelements 43 ohne grosse Phasenverschiebung folgen. Wenn die Viskosität des Harzsystems infolge einsetzender Gelierung steigt, steigt auch der Bewegungswiderstand. Diese hat zur Folge, dass die Koppelfedern 45 und 46 beim Auf- bzw. Abwärtshub des Treibelements 43 zunehmend komprimiert werden und sich der mittlere Schaltkontakt 63 auf den oberen bzw. unteren Schaltkontakt 61 bzw. 62 hin bewegt. Ab einer bestimmten Viskosität ist der Bewegungswiderstand schliesslich so gross, dass sich die betreffenden Schaltkontakte berühren und den Stromkreis schliessen. Welcher der beiden Kontakte 61 und 62 mit dem mittleren Kontakt 63 in Berührung kommt, hängt davon ab, welcher Bewegungshub im Moment des Erreichens der kritischen Viskositätsgrenze gerade ausgeführt wurde. Durch das Schliessen des einen bzw. anderen Stromkreises wird die Stoppuhr 6 angehalten und gleichzeitig, angedeutet durch die Steuerleitung 101, der Antriebsmotor 41 abgestellt. Ferner kann auch ein Signal an den Temperaturschreiber 5 gesandt werden, welcher den Zeitpunkt des Erreichens der Viskositätsgrenze in geeigneter Weise markiert (Steuerleitung 102).

Den eigentlichen Detektor für die Viskositätsgrenze bilden die federgekoppelten Schaltkontakte 61-63. Durch entsprechende Bemessung der Koppelfedern 45 und 46 und Abstimmung auf die Beschaffenheit des als Zähigkeitsfühler wirkenden Temperaturfühlers 3 ist erreicht, dass dieser Detektor genau bei einem Viskositätswert anspricht, der für das Eintreten der Gelierung charakteristisch ist. Ueblicherweise wird eine Schalt-Viskosität von etwa 2000 Pa s gewählt.

Der Temperaturfühler 3 ist ein dünnes zylindrisches Stahl-Rohr, an dessen unterem Ende das eigentliche temperatursensitive Element 31 sitzt. Es kann dies beispielsweise ein Fe-Const-Thermoelement sein. Vorzugsweise ist es mit dem Stahlmantel des Fühlers 3 verbunden, so dass es nur eine geringe Verzögerungszeit von vorzugsweise weniger als 1-2 s aufweist. Die elektrischen Anschlüsse des Elements 31 verlaufen durch das Innere des Stahlrohrs und sind mit dem Registriergerät 5 verbunden. Dieses zeichnet den zeitlichen Verlauf der Temperatur des Harzsystemes im Probenröhrchen 2 kontinuierlich auf.

Das Behältnis 2 für das zu untersuchende Harzsystem ist ein relativ dünnes Glasröhrchen mit einem Fassungsvermögen im Kubikzentimeterbereich, vorzugsweise rund 1 cm$^3$. Der innere Durchmesser des Röhrchens beträgt etwa 3-5 mm, seine Länge etwa 80-120 mm. In einem praktischen Beispiel ist das Glasröhrchen rund 100 mm lang mit einem inneren Durchmesser von 4 mm und einem äusseren Durchmesser von 6 mm. Der Fühler 3 besitzt einen Durchmesser von 1,5 mm, das eingefüllte Harzvolumen beträgt bei einer Füllhöhe von rund 80 mm etwa 1 cm$^3$. Das Proberöhrchen ist vorzugsweise nahezu vollständig in das Heizbad eingetaucht.

Durch die angegebene Bemessung des Probenbehältnisses 2 wird erreicht, dass praktisch über das gesamte Probenvolumen eine weitestgehend homogene Temperaturverteilung herrscht. Die Anordnung des thermosensitiven Elements 31 direkt am Viskositätsfühler bzw. die Verwendung eines Thermofühlers gleichzeitig auch als Viskositätsfühler erlaubt, dass die Harztemperatur am selben Ort gemessen wird, wo auch die Gelierung erfasst wird, und führt somit zu aussagekräftigeren und zuverlässigeren Ergebnissen als bei bekannten Geräten dieser Art.

Für optimale Messergebnisse hat es sich als vorteilhaft erwiesen, wenn die mittlere Bewegungsgeschwindigkeit des Zähigkeitsfühlers, hier also des als solcher wirkenden Temperaturfühlers 3, in der Grössenordnung von etwa einem halben Millimeter pro Sekunde liegt. Die Hubbewegung beträgt vorzugsweise 10-20 mm, wobei eine Zykluszeit (Zeit für einen vollständigen Aufwärts- und Abwärtshub) von 1 Minute oder grösser eingestellt sein sollte.

Die in der Fig. 1 obere Koppelfeder 45 ist zusätzlich zur Antriebskraft des Treibelements 43 auch noch mit dem Gewicht der Fühleranordnung belastet. Dadurch würden sich für den Aufwärtshub und den Abwärtshub verschiedene Schaltpunkte ergeben. Durch entsprechende Auslegung dieser Feder ist jedoch dafür gesorgt, dass die Kontakte 61-63 in beiden Bewegungshüben beim selben Viskositätswert schalten.

Nach dem Ansprechen des durch die Schaltkontakte 61-63 gebildeten Viskositätsdetektors stehen am Registriergerät der zeitliche Temperaturverlauf des untersuchten Harzsystemes während der Gelierung bei

4

der durch das Heizbad vorgegebenen Soll-umgebungstemperatur und an der Stoppuhr die bis zum Eintreten der Gelierung (definiert durch das Erreichen einer vorgegebenen Viskosität) verstrichene Gelierzeit zur Verfügung. Diese Temperatur- und Zeitdaten können nun zur Bestimmung des zeitlichen Gelierverhaltens gemäss dem nachstehend erläuterten erfindungsgemässen Verfahren entweder "von Hand" oder vorzugsweise automatisch oder halbautomatisch mittels der Datenverarbeitungseinrichtung 7 ausgewertet werden. Letztere ist vorzugsweise auch dazu eingerichtet, die übrigen Komponenten der Vorrichtung, wie z.B. die Temperaturregelung 14, die Stoppuhr 6, den Antriebsmotor 41 und das Registriergerät 5 zu steuern bzw. mit diesen zu kommunizieren. Selbstverständlich können der Temperaturfühler 3 und die Schaltkontakte 61-63 auch direkt an die Datenverarbeitungseinrichtung 7 angeschlossen sein, wenn diese mit entsprechenden Schnittstellen versehen ist. Die diversen Verbindungsgmöglichkeiten sind in der Fig. 1 durch nicht bezeichnete strichpunktierte Linien angedeutet. Ferner kann z.B. die Stoppuhr auch in die Datenverarbeitungseinrichtung integriert oder Bestandteil derselben oder Software-mässig realisiert sein.

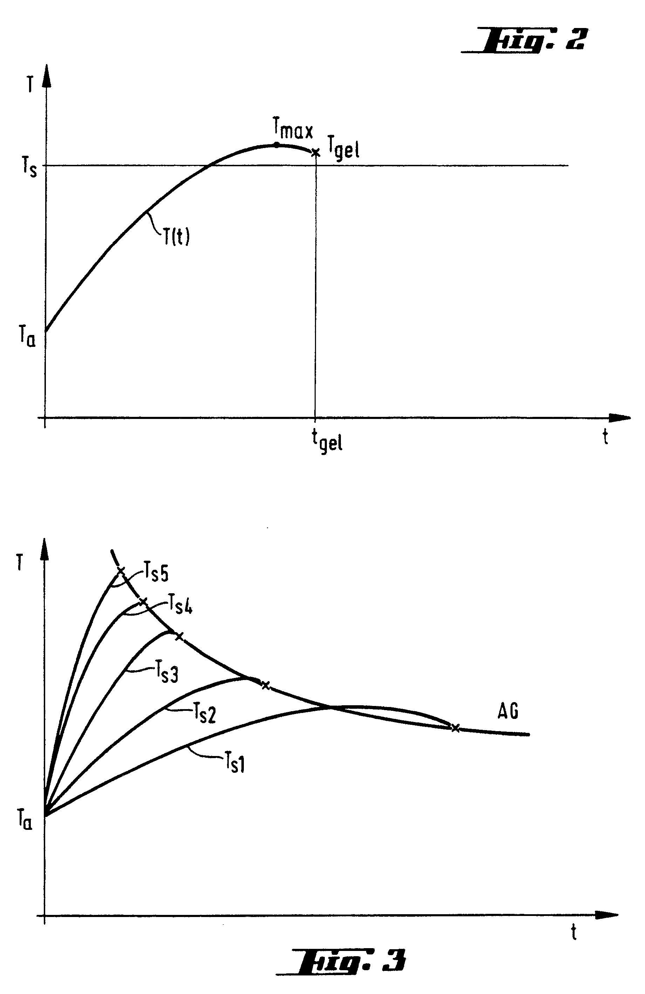

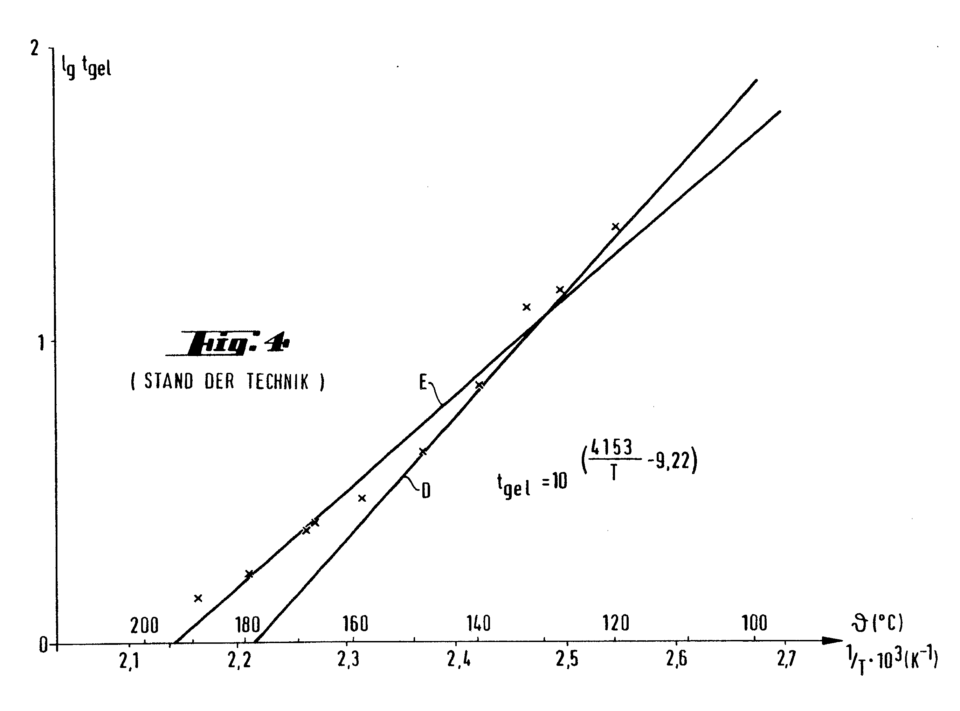

Fig. 2 stellt einen typischen Temperaturverlauf T(t) eines Harzsystems während der Gelierung bei einer durch das Heizbad vorgegebenen Soll-umgebungstemperatur $T_s$ dar. Wenn das harzgefüllte Probenbehältnis in das Heizbad eingetaucht wird, hat das Harz zunächst eine niedrigere gere Anfangstemperatur $T_a$. Diese steigt dann an und strebt einem Maximum $T_{max}$ zu, welches aufgrund der Exothermie des Harzsystems über der Soll-umgebungstemperatur $T_s$ liegt. Anschliessend sinkt die Temperatur wieder und nähert sich der Soll-umgebungstemperatur $T_s$; der ideale isotherme Verlauf wird also in der Praxis niemals erreicht. Irgendwo zwischen dem Punkt $T_{max}$ und dem asymptotischen Annähern an die Soll-umgebungstemperatur $T_s$ (oder je nach Soll-umgebungstemperatur auch schon vor dem Erreichen der Maximaltemperatur $T_{max}$, vgl. z.B. ungesättigte Polyester) wird der Gelierpunkt zur Zeit $t_{gel}$ erreicht. Die Harztemperatur zu diesem Zeitpunkt ist mit $T_{gel}$ bezeichnet.

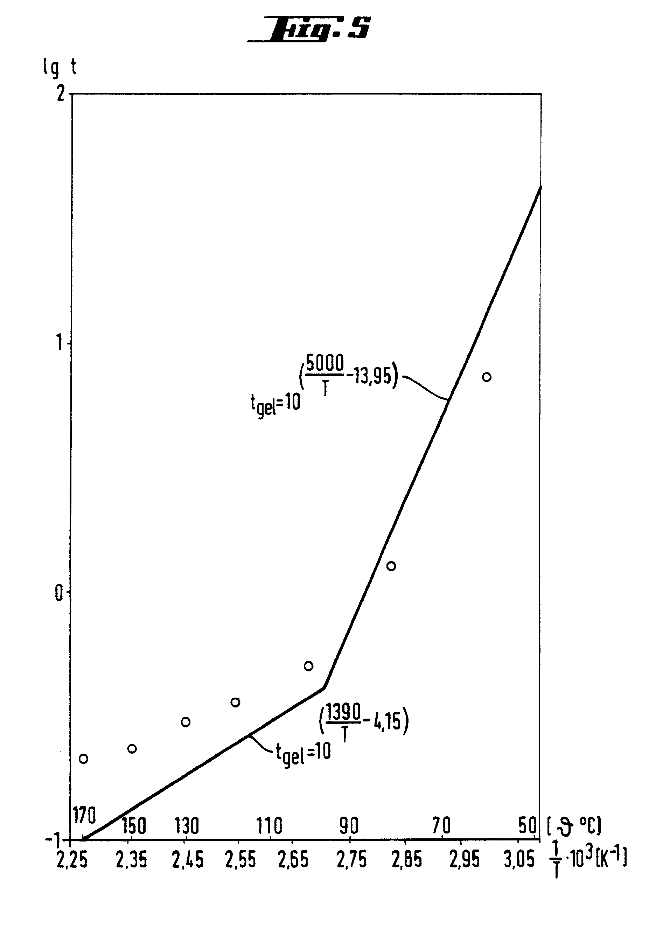

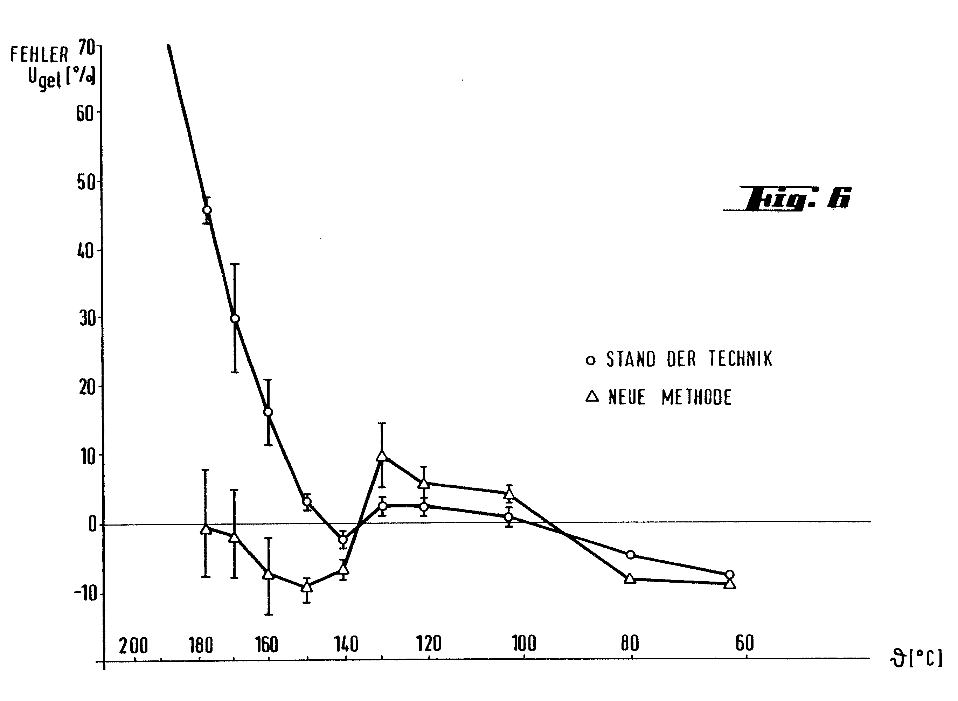

Fig. 3 zeigt mehrere für ein und dasselbe Harzsystem aufgenommene Temperaturverläufe T(t) für verschiedene vorgegebene Soll-umgebungstemperaturen $T_{s1}$ bis $T_{s5}$, wobei die Gelierpunkte jeweils mit einem x gekennzeichnet sind. Die Gelierpunkte sind im Idealfall durch eine Kurve AG verbunden, welche der Arrhenius-Gleichung

$$t_{gel}(T) = 10^{(\frac{a}{T} - b)}$$

genügt. Darin sind a und b Reaktionskonstanten, die das Harzsystem beschreiben und die zu bestimmen Ziel von Gelierzeituntersuchungen ist. Der Kleinbuchstabe t bedeutet hier und im folgenden immer die Variable Zeit, der Grossbuchstabe T die (absolute) Temperatur.

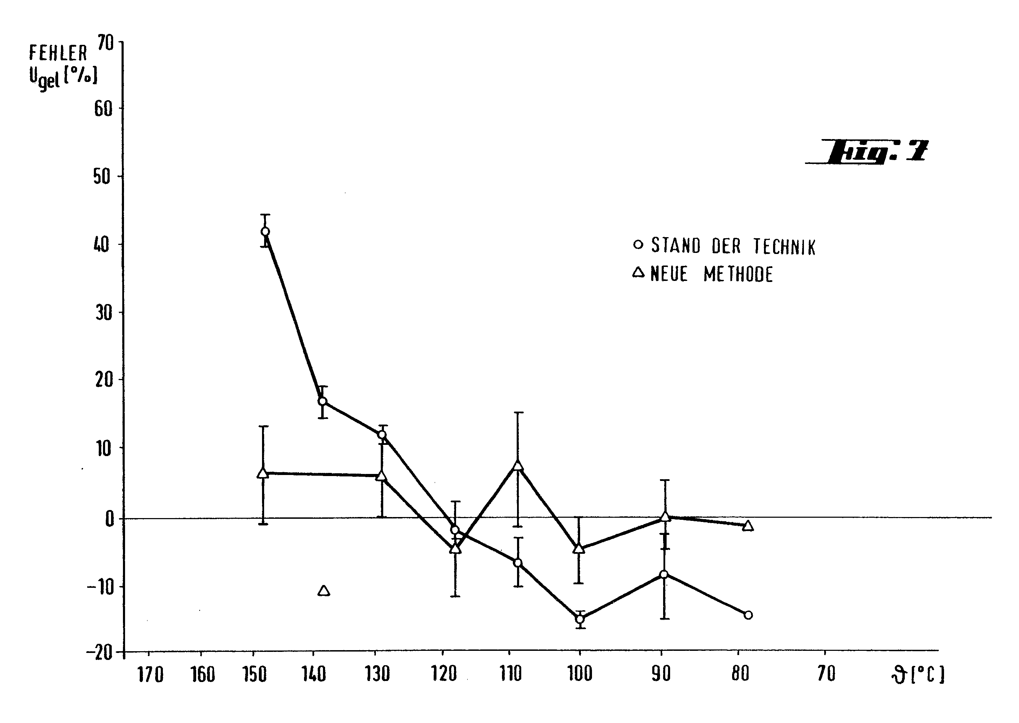

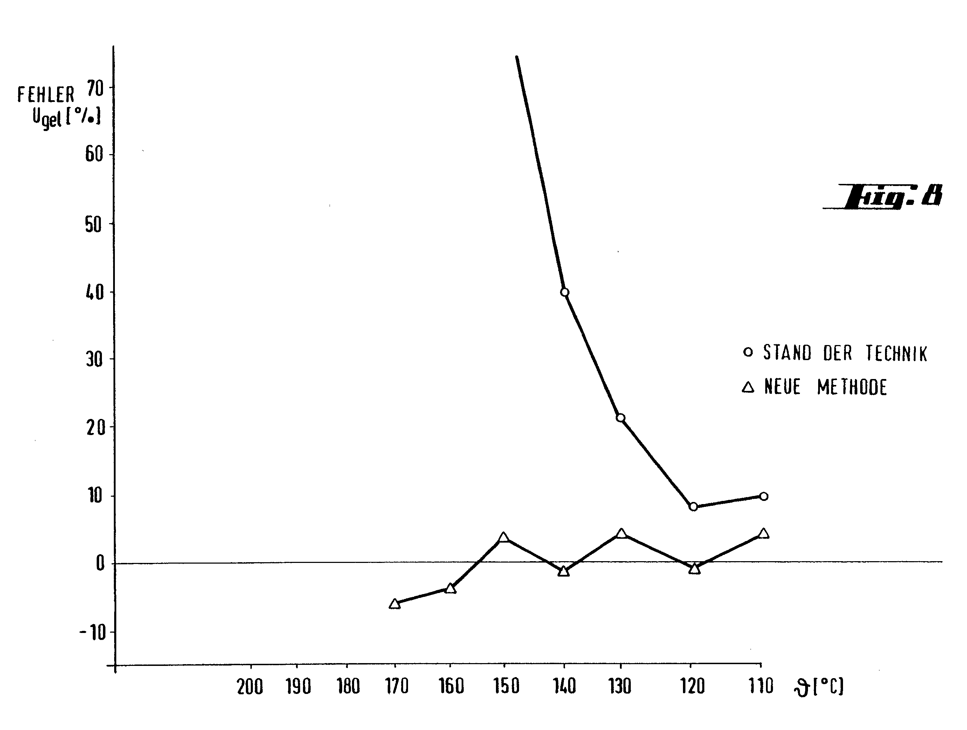

In einem einfach-logorithmischen Koordinatensystem $\lg t/T^{-1}$ ist die Arrhenius-Gleichung durch eine Gerade dargestellt, wobei die Reaktionskonstante a die Steigung und b den Ordinatenabschnitt ($\lg$ t-Achse) bedeuten. Trägt man die bei verschiedenen Soll-umgebungstemperaturen gefundenen Gelierpunkte (Wertepaare Gelierzeit $t_{gel}$/Geliertemperatur $T_{gel}$) in ein solches Koordinatensystem ein, so stellt man fest, dass die einzelnen Punkte in der Regel so streuen (z.B. Fig. 4), dass sich keine eindeutige Gerade durch sie definieren lässt. Auch die üblichen mathematischen Methoden der Regressions-Rechnung versagen hier in der Regel. Mit anderen Worten, auf diese Weise können die Reaktionskonstanten a und b der Arrhenius-Gleichung zumindest nicht mit der oftmals erwünschten hohen Genauigkeit ermittelt werden. Hier setzt nun das erfindungsgemässe Verfahren ein.

Das erfindungsgemässe Verfahren beschreitet einen völlig anderen Weg zur Bestimmung der Reaktionskonstanten: Zentraler Punkt des erfindungsgemässen Verfahrens ist der chemische Umsatz während der Gelierung. Zweckmässigerweise wird gleich mit dem normierten Umsatz gearbeitet, d.h., der Umsatz zum Gelierzeitpunkt wird als Eins angenommen.

Der Umsatz ist durch das Zeitintegral über die Reaktionsgeschwindigkeit über die jeweilige Zeitspanne gegeben. Die Reaktionsgeschwindigkeit ist verkehrt proportional der (temperaturabhängigen) Gelierzeit, so dass man nach Einsetzen der Arrhenius-Gleichung für den normierten Umsatz $U_{gel}$ schliesslich die Beziehung

$$U_{gel} = \int_0^{t_{gel}} \frac{1}{10^{(\frac{a}{T} - b)}} \cdot dt$$

erhält, in welcher die Temperatur T selbst eine Funktion der Zeit t darstellt. Wenn a und b für ein Harzsystem exakt bekannt sind und die Gelierzeit $t_{gel}$ für dieses System exakt gemessen wurde (theoretisch), so muss dieses Integral den Wert 1 ergeben.

Die Berechnung des vorstehenden Integrals ist analytisch praktisch nicht möglich, gemäss einem wesentlichen Aspekt der Erfindung aber anhand der gemessenen zeitlichen Temperaturverläufte T(t) leicht durchführbar. Diese Schritt sei jedoch zunächst zurückgestellt.

Gemäss dem Hauptgedanken des erfindungsgemässen Verfahrens werden nun für eine Mehrzahl von verschiedenen Soll-umgebungstemperaturen $T_s$ die Gelierzeiten $t_{gel}$ gemessen und dann für jede Soll-umgebungstemperatur mit willkürlich angenommenen Startwerten $a_o,b_o$ für die Reaktionskonstanten (für alle Solltemperaturen gleich) die Umsatzintegrale berechnet. Falls die Umsatzintegrale nicht alle den Idealwert 1 ergeben (was natürlich reiner Zufall wäre), werden die Reaktionskonstanten nun so lange variiert, bis die jeweils neu berechneten Umsatzintegrale innerhalb einer vorgegebenen Fehlergrenze (beispielsweise ±5%) beim Idealwert 1 liegen. Die Variation kann beispielsweise nach der bekannten Methode der kleinsten Quadrate erfolgen. Die schliesslich so gefundenen Reaktionskonstanten a und b beschreiben dann das untersuchte Harzsystem und stellen das Ergebnis des Verfahrens dar. Sie können selbstverständlich als Basis für weitere Berechnungen und Untersuchungen weiterverwendet werden.

Für die Praxis ist es vorteilhaft, als Startwerte $a_o,b_o$ für die Reaktionskonstanten nicht irgendwelche Werte zu benutzen, sondern diejenigen, die sich grob aus den gemessenen Gelierpunkten bei den verschiedenen Soll-umgebungstemperaturen ergeben. Wie schon erwähnt, ergeben sie sich als Steigung und Ordinatenabschnitt einer approximativ durch die Gelierpunkte gelegten Geraden im genannten halb-logarithmischen Koordinatennetz.

Nun zur numerischen Berechnung des Umsatz-Integrals. Man teilt dazu die Zeitachse in eine Vielzahl von gleichen Zeitintervallen $\Delta t$ ein und approximiert die gemessene Temperaturverlaufskurve bei der betreffenden Solltemperatur in jedem Intervall durch eine Gerade. Für das Integral ergibt sich dann der Näherungswert

$$U_{gel} \approx \sum_n \frac{\Delta t}{10^{(\frac{a}{T}_n - b)}} \Bigg|_o^{t_{gel}}$$

worin $\Delta t$ die Länge eines Zeitintervalls bzw. -inkrements ist, $T_n$ die gemessene Temperatur des Harzes zu einem Zeitpunkt n (z.B. Beginn eines Intervalls n) darstellt und die Summation über sämtliche Zeitintervalle von Beginn der Messung bis zum Erreichen des Gelierpunkts zu erfolgen hat. Diese Art der (näherungsweisen) Integration ist besonders an die automatische, rechnergestützte Messwerterfassung und -verarbeitung angepasst, da dort die zeitlichen Temperaturverläufe ohnehin nicht in Form eines geschlossenen Kurvenzugs, sondern in Form von üblicherweise in regelmässigen Abständen ermittelten einzelnen Messpunkten vorliegen. Selbstverständlich lässt sich die genannte Methode der stückweisen Approximation aber auch "von Hand" aus den registrierten Temperaturkurven durchführen.

Sogenannte latente Harzsysteme haben einen mehr oder weniger ausgeprägten Knickpunkt in der Arrhenius-Geraden. Genau genommen werden solche Systeme nicht durch eine einzige, sondern durch zwei verschiedene Geraden beschrieben. In solchen Fällen ist es zweckmässig, die vorstehenden Berechnungsschritte gruppenweise durchzuführen, d.h. die zu einer Arrhenius-Gleichung gehörenden Gelierpunkte getrennt von den der anderen Gleichung angehörigen Gelierpunkten auszuwerten, so dass schliesslich zwei getrennte Reaktionskonstantenpaare, a,b und a',b' als Ergebnis vorliegen. Welche Gelierpunkte zusammengehören, ist meist schon beim Bestimmen der Startwerte ersichtlich. Ein weiteres Indiz für das Vorhanden-

6

sein eines latenten Systems ist daran erkennbar, dass die Variation der Reaktionskonstanten für eine einzige Gerade nicht annehmbar konvergiert. In der Praxis bestimmt man die normierten Umsätze für die einzelnen Soll-umgebungstemperaturen und erkennte das Vorliegen eines latenten Systems daran, dass die Umsätze ab einer bestimmten Temperatur zunehmend deutlich nach oben vom erwarteten Verlauf abzuweichen beginnen. Man führt nun die Iteration einmal für die zu Soll-umgebungstemperaturen unterhalb dieser bestimmten Temperatur gehörenden Gelierpunkte und einmal für die darüberliegenden Gelierpunkte aus und erhält so zwei Arrhenius-Geraden. Ihr Schnittpunkt bestimmt dann den sog. Latenzpunkt des Systemes.

Die für die Variation der Reaktionskonstanten massgebliche Fehlergrenze der Umsatzintegrale hängt sehr von der verwendeten Messvorrichtung ab. Je präziser die Messvorrichtung arbeitet, desto enger kann die Fehlergrenze gesetzt werden. Die erfindungsgemässe Vorrichtung ist diesbezüglich besonders vorteilhaft und erlaubt Fehlergrenzen bis unter 2 %.

Bei bekannter Arrhenius-Funktion eines reaktiven Harzsystems kann duch Umkehrung der Rechnung bei bekanntem zeitlichen Temperaturverlauf der Gelierzeitpunkt vorausberechnet werden. Dies ermöglicht zum Beispiel eine Produktionsüberwachung durch Temperaturmessung in einem Formteil während seiner Herstellung. Auch bei der Konstruktion und Auslegung von Formteilen und Werkzeugen ist diese Rechnung von grosser Bedeutung und bildet einen wichtigen Ansatzpunkt zur Ausweitung von bekannten Computerprogrammen für die Thermoplast-Verarbeitung auf den Einsatz von reaktiven Systemen.

Ebenso kann auf der Grundlage des erfindungsgemässen Verfahrens der theoretische Ausgangswert in Schwunddiagrammen ermittelt werden, sofern der Schwund in der Anfangsphase der Reaktion proportional zum Umsatz ist. Man muss nur den Temperaturverlauf während der Schwundmessung oder unter Umständen in einem Parallelversuch unter gleichen Bedingungen mit aufzeichnen. Daraufhin rechnet man den Anteil es Gelierumsatzes bis zum Erreichen der Soll-umgebungstemperatur ( = Anteil des Schwundes) aus und addiert diesen zum gemessenen Schwund bis zum Gelierpunkt. Somit erhält man den Gesamtschwund vor dem Gelierpunkt. Der verbleibende Schwund ist der nur schwierig während der Härtung kompensierbare Schwund in fester Phase.

## Ansprüche

1. Verfahren zur Ermittlung des zeitlichen Gelierverhaltens eines reaktiven Harzsystemes durch Messung der Gelierzeiten bei verschiedenen Umgebungstemperaturen, bei dem man für eine Mehrzahl s von verschiedenen Soll-Umgebungstemperaturen $T_s$ die zeitlichen Verläufe $T(t)$ der Temperatur sowie die zugehörigen Gelierzeiten $t_{gel}$ einer Probe des Harzsystemes erfasst bzw. bestimmt und das zeitliche Gelierverhalten durch Auswertung der entspechenden Arrhenius-Geraden ermittelt, dadurch gekennzeichnet, dass man

   - für jede Soll-Umgebungstemperatur $T_s$ anhand des erfassten zeitlichen Temperaturverlaufs $T(t)$, der gemessenen Gelierzeit $t_{gel}$ und der das zeitliche Gelierverhalten beschreibenden Arrhenius-Gleichung

$$t_{gel}(T) = 10^{\left(\frac{a}{T} - b\right)}$$

mit willkürlich angenommenen Startwerten $a_o, b_o$ für die Reaktionskonstanten $a, b$ den vorzugsweise normierten Umsatz $U_{gel}$ bis zum Erreichen des Gelierpunkts berechnet, wobei die normierten Umsätze $U_{gel}$ gemäss der Beziehung

$$U_{gel} = \int_0^{t_{gel}} \frac{1}{10^{\left(\frac{a}{T} - b\right)}} \cdot dt$$

ermittelt werden, und worin a und b die Reaktionskonstanten, t die Intergrationsvariable Zeit, $t_{gel}$ die gemessene Gelierzeit und T die gemessene absolute Temperatur in Abhängigkeit der Zeit bedeuten, und

- die Reaktionskonstanten a,b bei der Berechnung aller Umsätze oder gruppenweise solange variiert, bis alle Umsätze bzw. die jeweils einer Gruppe angehörenden Umsätze innerhalb einer vorgegebenen Fehlergrenze liegen, wobei die auf diese Weise letztlich ermittelten Reaktionskonstanten das gesuchte Ergebnis darstellen.

2. Verfahren nach Anspruch 1, dadurch gekennzeichnet, dass die Startwerte $a_o, b_o$ der Reaktionskonstanten a,b aus den für die verschiedenen Soll-Umgebungstemperaturen $T_s$ gemessenen Gelierzeiten $t_{gel}$ ermittelt werden.

3. Verfahren nach Anspruch 1 oder 2, dadurch gekennzeichnet, dass man die normierten Umsätze $U_{gel}$ durch numerische Integration der erfassten zeitlichen Temperaturverläufe T(t) gemäss der Bezeichnung

$$U_{gel} = \sum_n \frac{\Delta t}{10^{(\frac{a}{T_n} - b)}} \Bigg|_o^{t_{gel}}$$

ermittelt, worin $T_n$ die bei der Soll-Umgebungstemperatur $T_s$ gemessene Temperatur der Probe zum Zeitpunkt n und $\Delta t$ das Zeitinkrement zwischen zwei benachbarten Zeitpunkten bedeuten.

4. Verfahren nach einem der Ansprüche 1-3, dadurch gekennzeichnet, dass die Fehlergrenze <5 % gesetzt wird.

5. Verfahren nach einem der Ansprüche 1-4, dadurch gekennzeichnet, dass die Variation der Reaktionskonstanten a,b iterativ z.B. nach der Methode der kleinsten Fehlerquadratsumme erfolgt.

6. Vorrichtung zur Bestimmung der Gelierzeit eines reaktiven Harzsystemes, mit
   - einem Behältnis (2) für eine Probe des zu untersuchenden Harzsystemes,
   - einem Heizorgan (1) für die im Behältnis (2) befindliche Probe,
   - einer Viskositätsmesseinrichtung (3, 4, 61-63) für die Probe,
   - einem Temperaturfühler (3, 31) zur Erfassung des tatsächlichen Temperaturverlaufes in der Probe und
   - einem Zeiterfassungsmittel (6),
   - wobei die Viskositätsmesseinrichtung (3, 4, 61-63) einen in das Probenbehältnis eintauchenden und in diesem automatisch bewegten Zähigkeitsfühler (3) sowie einen mit diesem Fühler zusammenwirkenden Detektor (61-63) umfasst, welcher anspricht, wenn die durch die Viskosität der Probe bewirkte, der Bewegung des Zähigkeitsfühlers (3) entgegenwirkende Widerstandskraft einen vorgegebenen Grenzwert überschreitet, und
   - wobei der Detektor (61-63) mit dem Zeiterfassungsmittel (6) zusammenwirkt, dadurch gekennzeichnet, dass
   - das temperatursensitive Element (31) des Temperaturfühlers auf dem Zähigkeitsfühler (3) und mit diesem mitbewegt angeordnet ist bzw. der Temperaturfühler selbst den Zähigkeitsfühler (3) bildet.

7. Vorrichtung nach Anspruch 6, dadurch gekennzeichnet, dass das Behältnis (2) für Probenvolumina im Bereich von etwa einem halben bis zu wenigen Kubikzentimetern ausgebildet ist.

8. Vorrichtung nach Anspruch 6 oder 7, dadurch gekennzeichnet, dass der Detektor (61-63) die der Bewegung des Zähigkeitsfühlers entgegenwirkende Widerstandskraft in jeder Bewegungsphase des Zähigkeitsfühlers überwacht.

9. Vorrichtung nach einem der Ansprüche 6-8, dadurch gekennzeichnet, dass das Heizorgan (1) ein thermostatisch geregeltes Bad, insbesondere Oelbad mit gegenüber dem Behältnis (2) bzw. der in diesem befindlichen Probe vergleichsweise sehr grosser Masse ist.

10. Vorrichtung nach einem der Ansprüche 6-9, dadurch gekennzeichnet, dass das Behältnis (2) als oben offenes, vorzugsweise zylindrisches Röhrchen mit gegenüber dem Durchmesser relativ grosser Länge ausgebildet ist.

11. Vorrichtung nach einem der Ansprüche 6-10, dadurch gekennzeichnet, dass der Zähigkeitsfühler als vorzugsweise zylindrischer Stab (3) ausgebildet und das temperatursensitive Element (31) des Temperaturfühlers am unteren Ende dieses Stabes angeordnet ist.

12. Vorrichtung nach einem der Ansprüche 6-11, dadurch gekennzeichnet, dass sie einen motorischen Antrieb (41) zum im wesentlichen vertikalen Auf- und Abbewegen des Zähigkeitsfühlers aufweist.

13. Vorrichtung nach Anspruch 12, dadurch gekennzeichnet, dass der Antrieb (41) ein auf- und abgehendes Treibelement (43) aufweist, welches über Koppelfedern (45, 46) kinematisch mit dem vorzugsweise geführten Zähigkeitsfühler (3) gekoppelt ist.

14. Vorrichtung nach Anspruch 13, dadurch gekennzeichnet, dass zwei einerseits mit dem Zähigkeitsfühler (3) und anderseits mit dem Treibelement (43) gekoppelte Schaltkontakte (61, 62 bzw. 63, 62) vorgesehen sind, welche umschalten, wenn die auf den Zähigkeitsfühler (3) wirkende Widerstandskraft während des einen bzw. des anderen Bewegungshubs des Fühlers einen Grenzwert übersteigt.

15. Vorrichtung nach Anspruch 14, dadurch gekennzeichnet, dass die Koppelfedern (45, 46) und die Schaltkontakte (61-63) so aufeinander abgestimmt sind, dass das Gewicht des Zähigkeitsfühlers (3) und der mit ihm unmittelbar verbundenen Teile kompensiert wird und beide Schaltkontakte beim Erreichen desselben Widerstandskraft-Grenzwertes umschalten.

16. Vorrichtung nach einem der Ansprüche 6-15, dadurch gekennzeichnet, dass sie eine elektronische Datenverarbeitungseinrichtung (7) zur wenigstens teilweise automatischen Auswertung der erfassten Temperatur-und Zeitdaten umfasst.

17. Vorrichtung nach einem der Ansprüche 6-16, dadurch gekennzeichnet, dass der Temperaturfühler (3) bzw. sein temperatursensitives Element (31) eine Ansprechverzögerung von max. 2 sec, vorzugsweise max. 1 sec aufweist.

18. Vorrichtung nach einem der Ansprüche 6-17, dadurch gekennzeichnet, dass die mittlere Bewegungsgeschwindigkeit des Zähigkeitsfühlers (3) in der Grössenordnung von etwa einem halben Millimeter pro Sekunde liegt.

19. Vorrichtung nach einem der Ansprüche 6-18, dadurch gekennzeichnet, dass der Zähigkeitsfühler (3) eine auf und ab gehende Hubbewegung von ca. 10-20 mm mit einer Zykluszeit von etwa 1 Minute oder grösser durchführt.

20. Vorrichtung nach einem der Ansprüche 6-19, dadurch gekennzeichnet, dass das Behältnis (2) für die Probe ein dünnwandiges Röhrchen mit einem inneren Durchmesser von ca. 3-5 mm und einer Länge von ca. 80-120 mm ist, und dass der Zähigkeitsfühler als Stab mit einem Durchmesser von ca. 1-2 mm ausgebildet ist.

## Claims

1. A method for determining the gelation behaviour with time of a reactive resin system by measuring the gelation times at different ambient temperatures, which comprises detecting or determining for a plurality s of different desired ambient temperatures $T_s$ the temperature curves with time $T(t)$ as well as the gelation times $t_{gel}$ pertaining thereto of a sample of the resin system, and ascertaining the gelation behaviour with time by evaluating the corresponding Arrhenius straight line, characterised in that
   - for each desired ambient temperature $T_s$ the preferably normalised conversion $U_{gel}$ until the gelation point is reached is calculated on the basis of the detected temperature curves with time T(t), the measured gelation time $t_{gel}$ and the Arrhenius equation describing the gelation behaviour with time:

$$t_{gel}(T) = 10 \cdot {}^{(\frac{a}{T} - b)}$$

using randomly chosen starting values $a_o$, $b_o$ for the reaction constants a, b, the normalised conversions $U_{gel}$ being determined on the basis of the relationship

$$U_{gel} = \int\limits_{0}^{t_{gel}} \frac{1}{10^{(\frac{a}{T} - b)}} \cdot dt$$

and wherein a and b represent the reaction constants, t represents the integration variable time, $t_{gel}$ represents the measured gelation time and T represents the measured absolute temperature as a function of time, and
- the reaction constants a, b are varied in the calculation of all conversions or group-wise until all the conversions or the conversions each belonging to one group fall within a pre-set range of error, the reaction constants ultimately arrived at in that manner representing the result sought.

2. A method according to claim 1, characterised in that the starting values $a_o$ $b_o$ of the reaction constants a, b are determined from the gelation times $t_{gel}$ measured for the various desired ambient temperatures $T_s$.

3. A method according to claim 1 or 2, characterised in that the normalised conversions $U_{gel}$ are determined by numerical integration of the detected temperature curves T(t) with time, on the basis of the designation

$$U_{gel} = \sum_{n} \frac{\Delta t}{10^{(\frac{a}{T_n} - b)}} \Bigg|_{o}^{t_{gel}}$$

wherein $T_n$ represents the temperature of the sample measured at the desired ambient temperature $T_s$ at the point in time n, and $\Delta t$ represents the time increment between two neighbouring points in time.

4. A method according to any one of claims 1 to 3, characterised in that an error range of <5% is permitted.

5. A method according to any one of claims 1 to 4, characterised in that the reaction constants a, b are varied iteratively, for example by the method of the least squares error sum.

6. An apparatus for determining the gelation time of a reactive resin system, comprising
- a receptacle (2) for a sample of the resin system to be investigated,
- a heating device (1) for the sample in the receptacle (2),
- a viscosity-measuring device (3, 4, 61-63) for the sample, ·
- a temperature sensor (3, 31) for measuring the actual temperature change in the sample, and
- a time-measuring device (6),
- the viscosity-measuring device (3, 4, 61-63) comprising a viscosity feeler (3) which dips into the sample receptacle and is moved automatically therein and a detector (61-63) which cooperates with that viscosity feeler and responds whenever the resisting force caused by the viscosity of the sample and resisting the movement of the viscosity feeler (3) exceeds a pre-set limit value, and
- the detector (61-63) cooperating with the time-measuring device (6), characterised in that
- the temperature-sensitive element (31) of the temperature sensor is arranged on the viscosity feeler (3) and is moved therewith or the temperature sensor itself forms the viscosity feeler (3).

7. An apparatus according to claim 6, characterised in that the receptacle (2) is dimensioned for receiving samples having a volume ranging approximately from half a cubic centimetre to a few cubic centimetres.

8. An apparatus according to claim 6 or 7, characterised in that the detector (61-63) monitors the resisting force resisting the movement of the viscosity feeler in every phase of movement of the viscosity feeler.

9. An apparatus according to any one of claims 6 to 8, characterised in that the heating device (1) is a thermostatically controlled bath, especially an oil bath of comparatively very great mass in relation to the receptacle (2) and the sample located therein.

10. An apparatus according to any one of claims 6 to 9, characterised in that the receptacle (2) is constructed as a small tube which is open at the top and preferably cylindrical and has a relatively great length in relation to the diameter.

11. An apparatus according to any one of claims 6 to 10, characterised in that the viscosity feeler is constructed as a preferably cylindrical rod (3) and the temperaturesensitive element (31) of the temperature sensor is arranged at the lower end of that rod.

12. An apparatus according to any one of claims 6 to 11, characterised in that it comprises a motor drive (41) for moving the viscosity feeler substantially vertically upward and downward.

13. An apparatus according to claim 12, characterised in that the drive (41) comprises a reciprocating driving element (43) which is kinematically coupled with the preferably guided viscosity feeler (3) via coupling springs (45, 46).

14. An apparatus according to claim 13, characterised in that two switching contacts (61, 62 and 63, 62) coupled on the one hand with the viscosity feeler (3) and on the other hand with the drive element (43) are provided, which switching contacts change over whenever the resisting force acting upon the viscosity feeler (3) exceeds a limit value during the one or the other movement stroke of the feeler.

15. An apparatus according to claim 14, characterised in that the coupling springs (45, 46) and the switching contacts (61-63) are adjusted with respect to each other in a manner such that the weight of the viscosity feeler (3) and any parts directly connected therewith is compensated and both switching contacts change over upon reaching the same limit value of the resisting force.

16. An apparatus according to any one of claims 6 to 15, characterised in that it comprises an electronic data processing unit (7) for at least partially automated evaluation of the temperature data and time data detected.

17. An apparatus according to any one of claims 6 to 16, characterised in that the temperature sensor (3) or its temperature-sensitive element (31) responds with a delay of at most 2 seconds, preferably at most 1 second.

18. An apparatus according to any one of claims 6 to 17, characterised in that the mean movement velocity of the viscosity feeler (3) is in the order of about half a millimetre per second.

19. An apparatus according to any one of claims 6 to 18, characterised in that the viscosity feeler (3) performs an upwardly and downwardly reciprocating movement of about 10 to 20 mm with a cycle of about 1 minute or greater.

20. An apparatus according to any one of claims 6 to 19, characterised in that the receptacle (2) for the sample is a small thin-walled tube having an internal diameter of about 3 to 5 mm and a length of about 80 to 120 mm, and the viscosity feeler is constructed as a rod having a diameter of about 1 to 2 mm.

## Revendications

1. Procédé de détermination du comportement de gélification en fonction du temps d'un système de résines réactif par mesure des temps de gélification à des températures ambiantes différentes, dans lequel on mesure, respectivement on détermine, pour une pluralité s de températures ambiantes de consigne $T_s$ différentes les évolutions dans le temps $T(t)$ de la température ainsi que les temps de gélification $t_{gel}$ respectifs d'un échantillon du système de résines et l'on détermine le comportement de gélification en fonction du temps par exploitation des droites d'Arrhenius correspondantes, caractérisé en ce que

   - pour chaque température ambiante de consigne $T_s$, on calcule la transformation $U_{gel}$ de préférence normalisée jusqu' à ce que soit atteint le point de gélification, à l'aide de l'évolution dans le temps de température $T(t)$ et du temps de gélification $T_{gel}$ mesurés, et de l'équation d'Arrhenius décrivant le comportement de gélification en fonction du temps

$$t_{gel}(T) = 10^{\left(\frac{a}{T} - b\right)}$$

avec des valeurs initiales $a_o, b_o$ quelconques des constantes de réaction $a, b$, les transformations normalisées $U_{gel}$ étant déterminée d'après la relation

$$U_{gel} = \int_0^{t_{gel}} \frac{1}{10^{\left(\frac{a}{T} - b\right)}} \cdot dt$$

et où $a$ et $b$ sont les constantes de réaction, $t$ la variable d'intégration temps, $t_{gel}$ le temps de gélification mesuré et $T$ la température absolue mesurée en fonction du temps, et

   - on fait varier les constantes de réaction $a, b$, lors du calcul de toutes les transformations ou par groupes, jusqu' à ce que toutes les transformations, ou les transformations appartenant chaque fois à un groupe, se situent à l'intérieur d'une limite d'erreur prédéterminée, les dernières constantes de réaction déterminées de cette façon représentant le résultat cherché.

2. Procédé selon la revendication 1, caractérisé en ce que les valeurs initiales $a_o, b_o$ des constantes de réaction $a, b$ sont déterminées à partir des temps de gélification $t_{gel}$ mesurés pour les différentes températures ambiantes de consigne $T_s$.

3. Procédé selon la revendication 1 ou 2, caractérisé en ce que l'on détermine les transformations normalisées $U_{gel}$ par intégration numérique des évolutions dans le temps de la température $T(t)$ suivant l'expression

$$U_{gel} = \sum_n \frac{\Delta t}{10^{\left(\frac{a}{T_n} - b\right)}} \cdot \Bigg|_{t_{gel}}$$

dans laquelle $T_n$ est la température de l'échantillon, mesurée pour la température ambiante de consigne $T_s$, à l'instant $n$ et $\Delta t$ est l'incrément du temps entre deux instants consécutifs.

4. Procédé selon l'une des revendications 1 à 3, caractérisé en ce que la limite d'erreur est posée <5%.

5. Procédé selon l'une des revendications 1 à 4, caractérisé en ce que la variation des constantes de réaction a,b s'effectue par itération, par exemple suivant la méthode de la plus petite somme des carrés des erreurs.

6. Dispositif pour la détermination du temps de gélification d'un système de résines réactif, comportant
   - un récipient (2) pour un échantillon du système de résines à étudier,
   - un organe de chauffage (1) pour l'échantillon se trouvant dans le récipient (2),
   - un dispositif de mesure de la viscosité (3,4,61 à 63) pour l'échantillon,
   - une sonde de température (3,31) pour la mesure de l'évolution effective de la température dans l'échantillon, et
   - un moyen de mesure du temps (6),
   - le dispositif de mesure de la viscosité (3,4,61 à 63) comprenant une sonde de viscosité (61 à 63) plongeant dans le récipient contenant l'échantillon et déplacée automatiquement dans celui-ci et un capteur coopérant avec ladite sonde et qui réagit lorsque, du fait de la viscosité de l'échantillon, la force de résistance qui s'oppose au mouvement de la sonde de viscosité (3) dépasse une valeur limite prédéterminée, et
   - le capteur (61 à 63) coopérant avec le moyen de mesure du temps (6), caractérisé en ce que
   - l'élément sensible à la température (31) de la sonde de température est disposé sur la sonde de viscosité (3) et se déplace avec elle, voire la sonde de température constitue la sonde de viscosité (3) elle-même.

7. Dispositif selon la revendication 6, caractérisé en ce que le récipient (2) est réalisé pour des volumes d'échantillon, dans la plage d'à peu près un demi à quelques centimètres cubes.

8. Dispositif selon la revendication 6 ou 7, caractérisé en ce que le capteur (61 à 63) surveille la force de résistance s'opposant au déplacement de la sonde de viscosité dans chaque phase de déplacement de la sonde de température.

9. Dispositif selon l'une des revendications 6 à 8, caractérisé en ce que l'organe de chauffage (1) est un bain réglé thermostatiquement, en particulier un bain d'huile avec une masse qui est comparativement très importante par rapport au récipient (2), ou à l'échantillon s'y trouvant.

10. Dispositif selon l'une des revendications 6 à 9, caractérisé en ce que le récipient (2) est réalisé sous la forme d'un petit tube avec une longueur relativement grande par rapport à son diamètre.

11. Dispositif selon l'une des revendications 6 à 10, caractérisé en ce que la sonde de viscosité est réalisée de préférence sous forme de tige (3) cylindrique et l'élément sensible à la température (31) de la sonde de température est disposé à l'extrémité inférieure de cette tige.

12. Dispositif selon l'une des revendications 6 à 11, caractérisé en ce qu'il présente un entraînement motorisé (41) pour déplacer la température dans un mouvement sensiblement vertical de montée et de descente.

13. Dispositif selon la revendication 12, caractérisé en ce que l'entraînement (41) présente un élément moteur (43) montant et descendant, qui est accouplé cinématiquement par des ressorts de couplage (45,46) à la sonde de viscosité (3) qui est de préférence guidée.

14. Dispositif selon la revendication 13, caractérisé en ce que deux contacts de commutation (61,62, et 63,62) sont prévus, couplés d'une part à la sonde de viscosité (3) et d'autre part à l'élément moteur (43), qui effectuent la commutation lorsque la force de résistance agissant sur la sonde de viscosité (3) pendant l'une ou l'autre course de déplacement de la sonde dépasse une valeur limite.

15. Dispositif selon la revendication 14, caractérisé en ce que les ressorts de couplage (45,46) et les contacts de commutation (61 à 63) sont accordés les uns par rapport aux autres de telle façon que le poids de la sonde de viscosité (3) et des éléments lui étant directement reliés est compensé et les deux contacts de commutation effectuent le commutation lorsque la valeur limite de la force de réaction de la sonde est atteinte.

**16.** Dispositif selon l'une des revendications 6 à 15, caractérisé en ce qu' il comprend un dispositif électronique de traitement des données (7) pour exploiter au moins partiellement automatiquement les valeurs de température et de temps mesurées.

**17.** Dispositif selon l'une des revendications 6 à 16, caractérisé en ce que la sonde de température (3), voire son élément sensible à la température (31) présente un retard de réaction de 2 secondes au maximum, de préférence de 1 seconde au maximum.

**18.** Dispositif selon l'une des revendications 6 à 17, caractérisé en ce que la vitesse de déplacement moyenne de la sonde de viscosité (3) est de l'ordre de grandeur d'à peu près un demi millimètre par seconde.

**19.** Dispositif selon l'une des revendications 6 à 18, caractérisé en ce que la sonde de viscosité (3) effectue un mouvement alternatif montant et descendant d'à peu près 10 à 20 mm, avec une durée de cycle d'à peu près 1 minute ou plus.

**20.** Dispositif selon l'une des revendications 6 à 19, caractérisé en ce que le récipient (2) pour l'échantillon est un petit tube à paroi mince présentant un diamètre intérieur d'à peu près 3 à 5 mm et une longueur d'à peu près 80 à 120 mm, et que la sonde de viscosité est réalisée sous forme de tige avec un diamètre d'à peu près 1 à 2 mm.

**Fig. 1**

Fig. 2

Fig. 3

**Fig. 4**
( STAND DER TECHNIK )

$$t_{gel} = 10^{\left(\frac{4153}{T} - 9,22\right)}$$

# Fig. 5

$$t_{gel} = 10^{\left(\frac{5000}{T} - 13,95\right)}$$

$$t_{gel} = 10^{\left(\frac{1390}{T} - 4,15\right)}$$

EP 0 259 252 B1

Fig. 6

o STAND DER TECHNIK

△ NEUE METHODE

FEHLER $U_{gel}$ [%]

$\vartheta$ [°C]

19

EP 0 259 252 B1

Fig. 1

Fig. 8

EP 0 259 252 B1